# EUROPEAN PATENT APPLICATION

(11) **EP 4 285 810 A1**
(43) Date of publication of application: **06.12.2023**
(21) Application number: 22745735.5
(22) Date of filing: 21.01.2022
(51) Int. Cl.: A61B 1/045

(54) **MEDICAL IMAGE PROCESSING DEVICE, METHOD, AND PROGRAM**

(30) Priority: 27.01.2021 JP 2021010915
(71) Applicant: FUJIFILM Corporation, Tokyo 106-8620 (JP)
(72) Inventor: KAMON, Shumpei, Ashigarakami-gun, Kanagawa 258-8538 (JP)
(74) Representative: Hughes, Andrea Michelle
(86) International application number: PCT/JP2022/002133
(87) International publication number: WO 2022/163514

(57) **Abstract**

Provided are a medical image processing apparatus, a medical image processing method, and a medical image processing program by which it is possible to automatically perform time measurement related to an examination state of time-series medical images. In a medical image processing apparatus including a processor, the processor is configured to perform: medical image acquisition processing for sequentially acquiring time-series medical images; examination state recognition processing for recognizing an examination state on the basis of the sequentially acquired medical images; time measurement processing for performing time measurement related to the examination state; time measurement control processing for controlling a behavior of the time measurement in accordance with a recognition result of the examination state; and report processing for causing a report unit to report information on a state of the time measurement to a user.

## Description

### BACKGROUND OF THE INVENTION

### 1. Field of the Invention

The present invention relates to a medical image processing apparatus, a medical image processing method, and a medical image processing program, and in particular, to a technique for reporting useful information to a user during observation of a medical image.

### 2. Description of the Related Art

In the related art, an endoscopic examination data recording system has been proposed that records, in an examination data storage unit, transitions of a type of irradiation light used in an endoscopic examination, a spray state of a drug sprayed in a patient's body, an implementation situation of a treatment implemented by a doctor, an imaging state of an image captured by an endoscope, and the like (JP2017-12666A).

In addition, the endoscopic examination data recording system described in JP2017-12666A calculates a use time of each type of irradiation light, the number of times of use of each type of irradiation light, an observation time while using each drug, the number of times of use of each drug, an implementation time of each treatment, the number of times of implementation of each treatment, and the like in the endoscopic examination, records the calculated data in the examination data storage unit, further calculates a use time of each type of irradiation light, the number of times of use of each type of irradiation light, an observation time while using each drug, the number of times of use of each drug, an implementation time of each treatment, the number of times of implementation of each treatment, and the like between two designated points in the endoscopic examination, and records the calculated data in the examination data storage unit.

Note that two points, which are a use start time and a use end time of observation light, are designated by an observation light button, two points, which are an observation start time and an observation end time using a drug, are designated by a drug button, and two points, which are an implementation start time and an implementation end time of a selected treatment, are designated by a treatment button.

In addition, the endoscopic examination data recording system described in JP2017-12666A includes a display control unit that causes a display unit to display, on a screen thereof, transitions of the acquired irradiation light type data, drug spray data, and treatment implementation data during the endoscopic examination.

### SUMMARY OF THE INVENTION

According to the endoscopic examination data recording system described in JP2017-12666A, transitions of respective states of the endoscopic examination are automatically recorded in time series, and thus, the endoscopic examination can be analyzed quantitatively and obj ectively.

However, in order to measure the use time of each type of irradiation light, the observation time while using each drug, and the implementation time of the treatment, it is necessary to designate the two points, which are the use start time and the use end time of the observation light, the two points, which are the observation start time and the observation end time using the drug, and the two points, which are the implementation start time and the implementation end time of the treatment, by operating the observation light button, the drug button, and the treatment button, respectively, and a burden of button operations is imposed on a doctor during the endoscopic examination.

In the endoscopic examination in which a complicated procedure is required, it is preferable to reduce such button operations as much as possible.

The present invention has been made in view of such circumstances, and an object thereof is to provide a medical image processing apparatus, a medical image processing method, and a medical image processing program by which it is possible to automatically perform time measurement related to an examination state of time-series medical images.

In order to achieve the above object, an invention according to a first aspect is a medical image processing apparatus including a processor, in which the processor is configured to perform: medical image acquisition processing for sequentially acquiring time-series medical images; examination state recognition processing for recognizing an examination state on the basis of the sequentially acquired medical images; time measurement processing for performing time measurement related to the examination state; time measurement control processing for controlling a behavior of the time measurement in accordance with a recognition result of the examination state; and report processing for causing a report unit to report information on a state of the time measurement to a user.

According to the first aspect of the present invention, the examination state is automatically recognized on the basis of the sequentially acquired medical images, and the behavior of the time measurement related to the examination state is controlled in accordance with the recognition result of the examination state, and thus, it is possible to reduce a user's load for the time measurement during an examination using the time-series medical images. In addition, since the report unit reports the information on the state of the time measurement to the user, for example, in a case where the automatic recognition of the examination state fails, or in a case where the examination state is automatically recognized at a timing different from the timing intended by the user, the user can notice that.

In a medical image processing apparatus according a second aspect of the present invention, in the report processing, the report unit is preferably caused to report the information on the state of the time measurement to the user only for a certain period based on a timing at which the state of the time measurement is changed. If the information on the state of the time measurement is reported over the certain period, the examination may be interrupted, or the information on the state of the time measurement (e.g., information indicating "start of measurement") may be significantly different from the current examination state (e.g., "during measurement").

In a medical image processing apparatus according to a third aspect of the present invention, in the report processing, a display unit functioning as the report unit is preferably caused to display information on a time measured in the time measurement processing in real time in a first display region of the display unit and to display the information on the state of the time measurement in a second display region of the display unit, the second display region being different from the first display region, or a speaker functioning as the report unit is preferably caused to report the information on the state of the time measurement as sound information. The information on the time measured may be the measured time itself or may be a bar graph or the like having a length corresponding to the measured time.

In a medical image processing apparatus according to a fourth aspect of the present invention, the information on the state of the time measurement preferably includes information of one or more of a start, an end, a pause, or a restart of the time measurement, and, in the time measurement control processing, control of one or more of the start, the end, the pause, or the restart of the time measurement in the time measurement processing is preferably performed.

In a medical image processing apparatus according to a fifth aspect of the present invention, the processor is preferably configured to perform reception processing for receiving the information on the state of the time measurement from a user operating unit, and, in the time measurement control processing, upon reception of the information on the state of the time measurement from the user operating unit, the behavior of the time measurement is preferably controlled by preferentially using the received information on the state of the time measurement instead of the recognition result obtained in the examination state recognition processing. Accordingly, for example, in a case where the automatic recognition of the examination state fails, the user manually gives an instruction related to the information on the state of the time measurement, thereby performing correct time measurement.

In a medical image processing apparatus according to a sixth aspect of the present invention, the processor is preferably configured to perform processing for storing, in a storage unit, a time measurement result obtained in the time measurement processing. Accordingly, the time taken for an examination or the like can be checked later. Note that the time taken for the examination serves as an index of the quality of screening.

In a medical image processing apparatus according to a seventh aspect of the present invention, the processor is preferably configured to perform processing for storing, in a storage unit, a time measurement result obtained in the time measurement processing, and, if a behavior of the time measurement control processing is controlled by a user operation on the user operating unit, change a method for storing the time measurement result in the storage unit in accordance with a situation of the controlled behavior. Accordingly, it is possible to identify whether the time measurement result is measured only by automatic recognition of the examination state or is corrected by a user operation due to an error in automatic recognition of the examination state.

In a medical image processing apparatus according to an eighth aspect of the present invention, in the examination state recognition processing, whether the examination state indicates that a treatment is being performed is preferably recognized on the basis of the sequentially acquired medical images. Accordingly, the treatment time can be measured.

In a medical image processing apparatus according to a ninth aspect of the present invention, in the examination state recognition processing, treatment tool detection processing for detecting a treatment tool is preferably performed on the basis of the sequentially acquired medical images, and whether the treatment is being performed is preferably recognized in accordance with a detection result of the treatment tool.

In a medical image processing apparatus according to a tenth aspect of the present invention, the medical images are endoscopic images.

In a medical image processing apparatus according to an eleventh aspect of the present invention, in the examination state recognition processing, whether an endoscope is being inserted, is being removed, or is outside a body is preferably recognized on the basis of the sequentially acquired medical images.

In a medical image processing apparatus according to a twelfth aspect of the present invention, in the examination state recognition processing, landmark detection processing for detecting a landmark in a lumen is preferably performed on the basis of the sequentially acquired medical images, and the examination state is preferably recognized in accordance with a detection result of the landmark.

In a medical image processing apparatus according to a thirteenth aspect of the present invention, in the examination state recognition processing, movement detection processing for detecting a movement of the endoscope is preferably performed on the basis of the sequentially acquired medical images, and the examination state is preferably recognized in accordance with a detection result of the movement of the endoscope. If the state of the movement of the endoscope is recognized, it can be determined that the endoscope is being inserted if the endoscope is moving forward, and that the endoscope is being removed if the endoscope is moving backward. Thus, the timing at which the insertion transitions to the removal can be determined as the removal start.

An invention according to a fourteenth aspect is a medical image processing method including: a step of sequentially acquiring time-series medical images; an examination state recognition step of recognizing information on an examination state on the basis of the sequentially acquired medical images; a time measurement step of performing time measurement related to the examination state; a time measurement control step of controlling a behavior of the time measurement in accordance with a recognition result of the examination state; and a report step of causing a report unit to report information on a state of the time measurement to a user, in which a processor is configured to execute processing in each step.

In a medical image processing method according a fifteenth aspect of the present invention, in the report step, the report unit is preferably caused to report the information on the state of the time measurement to the user only for a certain period based on a timing at which the state of the time measurement is changed.

In a medical image processing method according to a sixteenth aspect of the present invention, in the report step, a display unit functioning as the report unit is preferably caused to display information on a time measured in the time measurement step in real time in a first display region of the display unit and to display the information on the state of the time measurement in a second display region of the display unit, the second display region being different from the first display region, or a speaker functioning as the report unit is preferably caused to report the information on the state of the time measurement as sound information.

A medical image processing method according to a seventeenth aspect of the present invention preferably includes a step of receiving the information on the state of the time measurement from a user operating unit, in which, in the time measurement control step, upon reception of the information on the state of the time measurement from the user operating unit, the behavior of the time measurement is preferably controlled by preferentially using the received information on the state of the time measurement instead of the recognition result obtained in the examination state recognition step.

An invention according to an eighteenth aspect is a medical image processing program for causing the processor to execute processing in each step in the medical image processing method according to any one of the fourteenth aspect to the seventeenth aspect of the present invention.

According to the present invention, it is possible to automatically perform time measurement related to an examination state of time-series medical images, and to reduce a load on a user during an examination.

### BRIEF DESCRIPTION OF THE DRAWINGS

Fig. 1 is a schematic diagram illustrating an overall configuration of an endoscope system including a medical image processing apparatus according to the present invention;
Fig. 2 is a block diagram illustrating an embodiment of the medical image processing apparatus;
Fig. 3 is a diagram illustrating a first report example by using a display;
Figs. 4A and 4B are diagrams illustrating a second report example by using the display;
Fig. 5 is a diagram illustrating an example of a screen displayed on the display when a measurement result is stored; and
Fig. 6 is a flowchart illustrating an embodiment of a medical image processing method according to the present invention.

### DESCRIPTION OF THE PREFERRED EMBODIMENTS

Hereinafter, preferred embodiments of a medical image processing apparatus, a medical image processing method, and a medical image processing program according to the present invention will be described with reference to the accompanying drawings. Overall Configuration of Endoscope System Including Medical Image Processing Apparatus

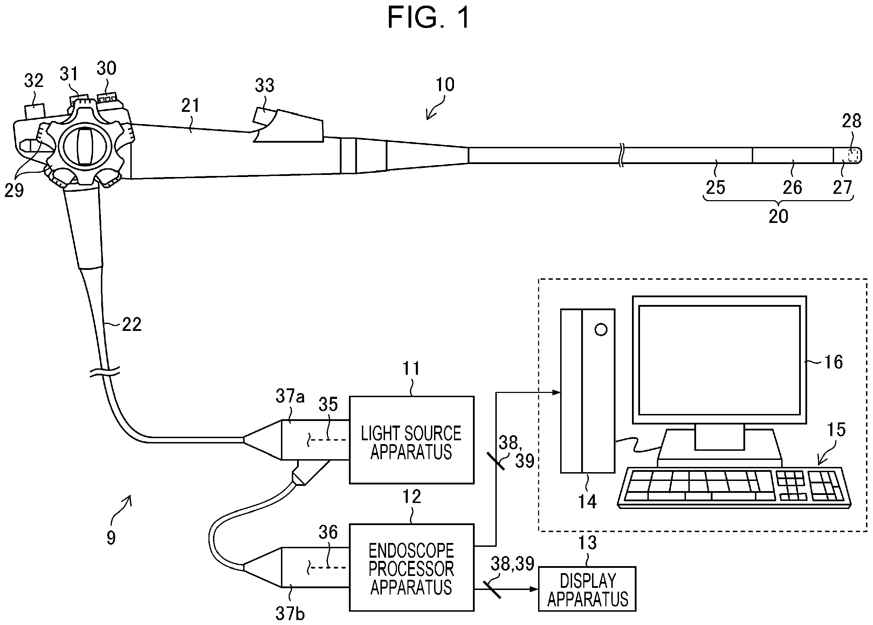

Fig. 1 is a schematic diagram illustrating an overall configuration of an endoscope system including a medical image processing apparatus according to the present invention.

As illustrated in Fig. 1, an endoscope system 9 includes an endoscope 10, which is an electronic endoscope, a light source apparatus 11, an endoscope processor apparatus 12, a display apparatus 13, a medical image processing apparatus 14, an operating unit 15, and a display 16.

The endoscope 10 captures time-series medical images including a subject image, and is, for example, a lower gastrointestinal tract scope. The endoscope 10 has an insertion part 20 to be inserted into a subject (large intestine, which is a luminal organ, in this example) and having a distal end and a proximal end, a handheld operating unit 21 provided continuously from the proximal end side of the insertion part 20 and grasped by a doctor who is a surgeon to perform various operations, and a universal cord 22 provided continuously from the handheld operating unit 21.

The entire insertion part 20 is formed to have a small diameter and an elongated shape. The insertion part 20 is constituted by continuously providing, in order from the proximal end side to the distal end side thereof, a soft part 25 having flexibility, a bending part 26 that can be bent by an operation of the handheld operating unit 21, and a tip part 27 in which an imaging optical system (objective lens), which is not illustrated, an imaging element 28, and the like are incorporated.

The imaging element 28 is an imaging element of a complementary metal oxide semiconductor (CMOS) type or a charge coupled device (CCD) type. Image light of a part to be observed is incident on an imaging surface of the imaging element 28 through an observation window and the objective lens. The observation window, which is not illustrated, is open on a distal end surface of the tip part 27, and the objective lens, which is not illustrated, is disposed behind the observation window. The imaging element 28 captures the image light of the part to be observed, which is incident on the imaging surface (converts the image light into an electric signal) and outputs an image signal.

The handheld operating unit 21 is provided with various operating members that are operated by a doctor (user). Specifically, the handheld operating unit 21 is provided with two types of bending operation knobs 29 to be used for a bending operation of the bending part 26, an air/water supply button 30 for air supply/water supply operations, and a suction button 31 for a suction operation. The handheld operating unit 21 is further provided with a still image capturing instruction unit 32 for issuing an instruction for capturing a still image 39 of the part to be observed and a treatment tool introduction port 33 for inserting a treatment tool (not illustrated) into a treatment tool insertion path (not illustrated) that penetrates through the insertion part 20.

The universal cord 22 is a connection cord for connecting the endoscope 10 to the light source apparatus 11. The universal cord 22 contains a light guide 35 that penetrates through the insertion part 20, a signal cable 36, and a fluid tube (not illustrated). In addition, an end portion of the universal cord 22 is provided with a connector 37a to be connected to the light source apparatus 11 and a connector 37b branching off from the connector 37a and to be connected to the endoscope processor apparatus 12.

By the connector 37a being connected to the light source apparatus 11, the light guide 35 and the fluid tube (not illustrated) are inserted into the light source apparatus 11. Thus, through the light guide 35 and the fluid tube (not illustrated), necessary illumination light, water, and gas are supplied from the light source apparatus 11 to the endoscope 10. As a result, the part to be observed is irradiated with the illumination light from an illumination window (not illustrated) on the distal end surface of the tip part 27. In accordance with a pressing operation on the above-described air/water supply button 30, the gas or water is injected from an air/water supply nozzle (not illustrated) on the distal end surface of the tip part 27 to the observation window (not illustrated) on the distal end surface.

By the connector 37b being connected to the endoscope processor apparatus 12, the signal cable 36 is electrically connected to the endoscope processor apparatus 12. Thus, through the signal cable 36, an image signal of the part to be observed is output from the imaging element 28 of the endoscope 10 to the endoscope processor apparatus 12, and also, a control signal is output from the endoscope processor apparatus 12 to the endoscope 10.

The light source apparatus 11 supplies the illumination light through the connector 37a to the light guide 35 of the endoscope 10. As the illumination light, light in various wavelength ranges is selected in accordance with an observation purpose, such as white light (light in a white wavelength range or light in a plurality of wavelength ranges), light in one or more specific wavelength ranges, or a combination thereof.

The endoscope processor apparatus 12 controls operations of the endoscope 10 through the connector 37b and the signal cable 36. In addition, based on the image signal acquired from the imaging element 28 of the endoscope 10 through the connector 37b and the signal cable 36, the endoscope processor apparatus 12 generates images (also referred to as "moving image 38") formed of time-series frame images 38a including the subject image. Furthermore, if the still image capturing instruction unit 32 is operated in the handheld operating unit 21 of the endoscope 10, concurrently with the generation of the moving image 38, the endoscope processor apparatus 12 sets one frame image in the moving image 38 as the still image 39 in accordance with the timing of the image capturing instruction.

The moving image 38 and the still image 39 are medical images obtained by capturing images of the inside of the subject, that is, a living body. In addition, if the moving image 38 and the still image 39 are images obtained with the above-described light in the specific wavelength range(s) (special light), both are special-light images. Furthermore, the endoscope processor apparatus 12 outputs the generated moving image 38 and the still image 39 to each of the display apparatus 13 and the medical image processing apparatus 14.

Note that the endoscope processor apparatus 12 may generate (acquire) the special-light image having information on the above-described specific wavelength range(s), on the basis of a usual-light image obtained with the above-described white light. In this case, the endoscope processor apparatus 12 functions as a special-light image acquisition unit. Then, the endoscope processor apparatus 12 obtains a signal in the specific wavelength range(s) by performing calculation based on color information of red, green, and blue [RGB] or cyan, magenta, and yellow [CMY] included in the usual-light image.

On the basis of, for example, at least one of the usual-light image obtained with the above-described white light or the special-light image obtained with the above-described light in the specific wavelength range(s) (special light), the endoscope processor apparatus 12 may generate a feature quantity image such as a known oxygen saturation image. In this case, the endoscope processor apparatus 12 functions as a feature quantity image generation unit. Note that each of the moving image 38 and the still image 39 including the above-described in-living-body image, the usual-light image, the special-light image, and the feature quantity image is a medical image obtained by converting results of imaging or measuring of a human body into an image for the purpose of image diagnosis or inspection.

The display apparatus 13 is connected to the endoscope processor apparatus 12 and functions as a display unit that displays the moving image 38 and the still image 39 input from the endoscope processor apparatus 12. A doctor (user) operates the insertion part 20 back and forth, for example, while viewing the moving image 38 displayed on the display apparatus 13, and, if a lesion or the like is found at the part to be observed, the doctor (user) operates the still image capturing instruction unit 32 to capture a still image of the part to be observed for diagnosis, biopsy, or the like.

### Medical Image Processing Apparatus

The medical image processing apparatus 14 recognizes an examination state on the basis of time-series medical images, controls a behavior of time measurement related to the examination state in accordance with a recognition result of the examination state, and reports information on a state of the time measurement to a user, and, for example, a personal computer is used as the medical image processing apparatus 14 in this embodiment. In addition, in addition to a keyboard, a mouse, or the like connected to the personal computer via wired or wireless connection, the operating unit 15 includes buttons provided in the handheld operating unit 21 of the endoscope 10, and various monitors, such as a liquid crystal monitor that can be connected to the personal computer, are used as the display (display unit) 16.

### Embodiment of Medical Image Processing Apparatus 14

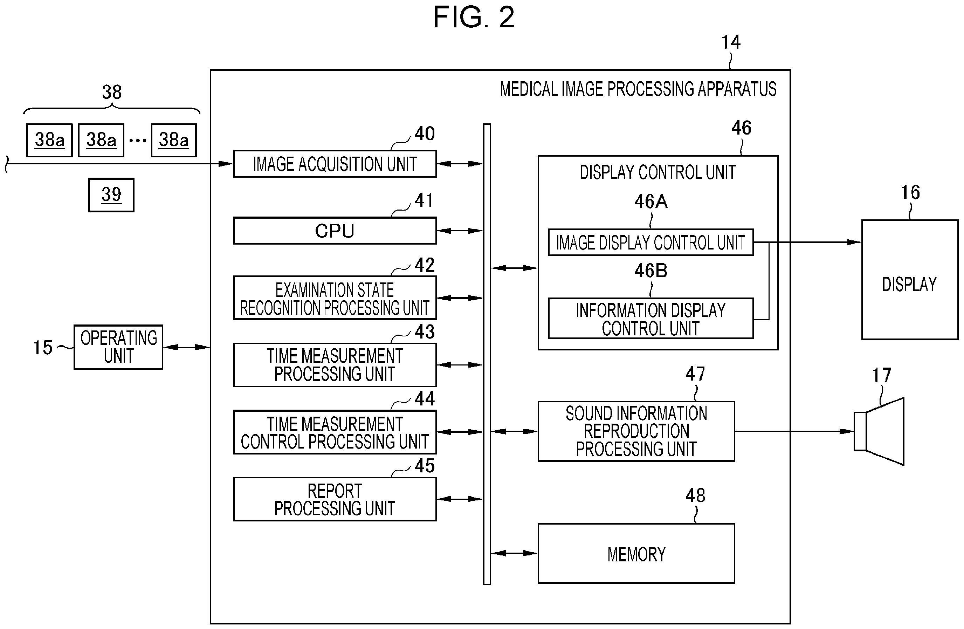

Fig. 2 is a block diagram illustrating an embodiment of the medical image processing apparatus.

The medical image processing apparatus 14 illustrated in Fig. 2 is constituted by a medical image acquisition unit 40, a central processing unit (CPU) 41, an examination state recognition processing unit 42, a time measurement processing unit 43, a time measurement control processing unit 44, a report processing unit 45, a display control unit 46, a sound information reproduction processing unit 47, and a memory 48, and processing of each unit is implemented by one or more processors.

The CPU 41 operates on the basis of various programs including an operation system and a medical image processing program according to the present invention that are stored in the memory 48, generally controls the medical image acquisition unit 40, the examination state recognition processing unit 42, the time measurement processing unit 43, the time measurement control processing unit 44, the report processing unit 45, the display control unit 46, and the sound information reproduction processing unit 47, and functions as some of these units.

The medical image acquisition unit 40 is a medical image acquisition processing unit that sequentially acquires, from the endoscope processor apparatus 12 (Fig. 1), the time-series frame images 38a including a subject image as medical images by using an image input/output interface, which is not illustrated, connected to the endoscope processor apparatus 12 via wired or wireless connection. In this example, the moving image 38 captured by the endoscope 10 is acquired. In addition, if the above-described still image 39 is captured while the moving image 38 is being captured by the endoscope 10, the medical image acquisition unit 40 acquires the moving image 38 and the still image 39 from the endoscope processor apparatus 12.

The examination state recognition processing unit 42 is a part that recognizes an examination state on the basis of the moving image 38 acquired by the medical image acquisition unit 40.

As an example of the recognition of the examination state, the examination state recognition processing unit 42 performs landmark detection processing for detecting an intraluminal landmark on the basis of the moving image 38.

This example is an embodiment in a case where the endoscope 10 is inserted into a large intestine and the large intestine is subjected to an endoscopic examination. In an endoscopic examination of the large intestine, a removal time (time required for insertion to the cecum and then return to the rectum), which is a time required for the examination, serves as an index of the quality of screening, and thus, the removal time is measured, and the measurement result is recorded.

In a case of the endoscopic examination of the large intestine, the examination state recognition processing unit 42 detects the Bauhin's valve in the ileocecal part of the large intestine, which is the boundary part between the large intestine and the small intestine, as an intraluminal landmark. In accordance with a detection result of the landmark (Bauhin's valve), it is recognized whether the distal end of the insertion part 20 of the endoscope 10 has reached an examination start position (examination state) in a case of the endoscopic examination of the large intestine. The detection of the intraluminal landmark based on the medical images can be performed by, for example, a known artificial intelligence (AI) technique.

In addition, as another example of the recognition of the examination state, the examination state recognition processing unit 42 recognizes that the endoscope 10 is being inserted, is being removed, or is outside the body on the basis of the moving image 38.

In a case where screening is performed while the endoscope 10 is being removed, a timing at which the endoscope 10 is switched from being inserted to being removed can be set as the timing of the start of removal (the start of measurement of the removal time). In addition, if the endoscope that is being removed comes out of the body, the measurement of the removal time can be ended.

As for whether the endoscope 10 is being inserted or removed, for example, if it is determined that the distal end of the endoscope 10 is moving forward on the basis of a change between frames in the size or position of an image in a partial region within the frame images 38a constituting the moving image 38, it can be recognized that the endoscope 10 is being inserted, and if it is determined that the distal end of the endoscope 10 is returning, it can be recognized that the endoscope 10 is being removed.

Furthermore, whether the distal end of the insertion part 20 of the endoscope 10 is present in a lumen or outside the body can be recognized from a feature quantity of the image, or can be recognized from a large change in the subject, a change in color, or the like of the frame image 38a. Then, if the recognition result transitions from the inside of the lumen to the outside of the body, the examination state recognition processing unit 42 can determine that the removal has ended (the examination has ended).

Furthermore, as still another example of the recognition of the examination state, the examination state recognition processing unit 42 recognizes whether the examination state indicates that a treatment is being performed on the basis of the moving image 38. For example, the examination state recognition processing unit 42 performs treatment tool detection processing for detecting a treatment tool on the basis of the moving image 38 and recognizes whether a treatment is being performed on the basis of a treatment tool detection result. That is, if a treatment tool is detected from the moving image 38, it can be recognized that the treatment is being performed during the detection period.

In addition, a timing at which the treatment tool is initially detected from the moving image 38 can be recognized as the start of the treatment, and a timing at which the treatment tool is no longer detected from the moving image 38 after being detected can be recognized as the end of the treatment.

Note that the end of the treatment may be recognized from the procedure of the treatment. For example, if the treatment tool is biopsy forceps, the timing at which the biopsy forceps are closed may be recognized as the end of the treatment, and if the treatment tool is endoscopic mucosal resection (EMR) or endoscopic submucosal dissection (ESD), the timing at which a clip treatment is completed may be recognized as the end of the treatment.

The time measurement processing unit 43 is a part that performs time measurement related to an examination state and measures a specific time during an examination.

As the specific time measured by the time measurement processing unit 43, the following two times can be given.

### 1. Removal Time in Endoscopic Examination of Large Intestine

It has been reported that if the removal time from the arrival at the cecum until the end of the examination is short, a lesion is more likely to be missed, and thus, the removal time is an index of the quality of screening.

### 2. Treatment Time for Therapy Etc.

The time required for the treatment serves as a reference for a patient's burden and for sedative administration.

In accordance with the recognition result of the examination state recognized by the examination state recognition processing unit 42, the time measurement control processing unit 44 controls the behavior of the time measurement by the time measurement processing unit 43. The information on the state of the time measurement by the time measurement processing unit 43 includes information of one or more of a start, an end, a pause, and a restart of the time measurement, and the time measurement control processing unit 44 controls the start, the end, and the like of the time measurement by the time measurement processing unit 43.

If the time measurement processing unit 43 measures the removal time in an endoscopic examination of the large intestine, the time measurement control processing unit 44 can cause the time measurement processing unit 43 to start the time measurement at a timing at which the predetermined landmark (Bauhin's valve) is detected by the examination state recognition processing unit 42 and then the Bauhin's valve is not detected, as a removal start timing, and can cause the time measurement processing unit 43 to end the time measurement at a timing at which the outside of the body is initially recognized by the examination state recognition processing unit 42, as a removal end timing.

In addition, if the examination state recognition processing unit 42 recognizes that the endoscope 10 is being inserted and then recognizes that the endoscope 10 is being removed, the time measurement control processing unit 44 may cause the time measurement processing unit 43 to start the time measurement at a timing at which the insertion is switched to the removal, as the removal start timing.

In a case where the time measurement processing unit 43 measures a treatment time for therapy or the like, if the examination state recognition processing unit 42 detects a treatment tool and recognizes that treatment is being performed, the time measurement control processing unit 44 causes the time measurement processing unit 43 to start the time measurement a timing at which the treatment tool is initially detected, as a treatment start timing, and causes the time measurement processing unit 43 to end the time measurement a timing at which the treatment tool is no longer detected for the first time after it is recognized by the examination state recognition processing unit 42 that the treatment is being performed, as a treatment end timing.

In addition, the CPU 41 performs reception processing for receiving information on the state of the time measurement from the operating unit 15, which is a user operating unit, upon reception of the information on the state of the time measurement from the operating unit 15, it is preferable that the time measurement control processing unit 44 preferentially use the received information on the state of the time measurement instead of the recognition result obtained in the examination state recognition processing unit 42 to control the behavior of the time measurement.

In a case where the examination state recognition processing unit 42 recognizes the information on the state of the time measurement, if a recognition error occurs, the behavior of the time measurement cannot be controlled correctly (the time measurement cannot be performed correctly), and thus, if the information on the state of the time measurement is received by a user operation, the time measurement control processing unit 44 uses the information on the state of the time measurement by a user operation preferentially over the recognition result obtained in the examination state recognition processing unit 42.

The report processing unit 45 is a part that reports the information on the state of the time measurement in the time measurement processing unit 43 by using at least one of the display 16 or a speaker 17 functioning as a report unit. Details of the report of the information on the state of the time measurement reported by the report processing unit 45 will be described later.

The display control unit 46 has an image display control unit 46A and an information display control unit 46B. The image display control unit 46A generates image data to be displayed on the basis of the medical images (the moving image 38) acquired by the medical image acquisition unit 40 and outputs the image data to the display 16. The information display control unit 46B generates image data to be displayed indicating information on the state of the time measurement on the basis of the information reported by the report processing unit 45 and outputs the image data to the display 16.

The display control unit 46 combines the image data of the moving image 38 output from the image display control unit 46A and the image data indicating the information on the state of the time measurement output from the information display control unit 46B, and outputs the combined image data to the display 16, thereby causing the display 16 to display (report) the information on the state of the time measurement together with the medical images.

On the basis of the information input from the report processing unit 45, the sound information reproduction processing unit 47 causes the speaker 17 to reproduce (report) sound information indicating the information on the state of the time measurement. Note that the sound information reproduction processing unit 47 can store a plurality of pieces of sound information in accordance with the content of the information on the state of the time measurement, select sound information corresponding to the information input from the report processing unit 45, and output the selected sound information to the speaker 17.

The memory 48 includes a flash memory, a read-only memory (ROM), a random access memory (RAM), a hard disk apparatus, and the like. The flash memory, the ROM, and the hard disk apparatus are non-volatile memories that store an operation system, various programs such as a medical image processing program according to the present invention, measured times (removal time and treatment time) measured by the time measurement processing unit 43 in association with a diagnosis report or the like, the captured still image 39, and the like. In addition, the RAM is a volatile memory from which data can be read and on which data can be written at high speed and that functions as an area for temporarily storing various programs stored in the non-volatile memory and as a work area for the CPU 41.

### Report of Information on State of Time Measurement

Next, the information on the state of the time measurement reported to the user by the report processing unit 45 via the display 16 or the like will be described.

### First Report Example

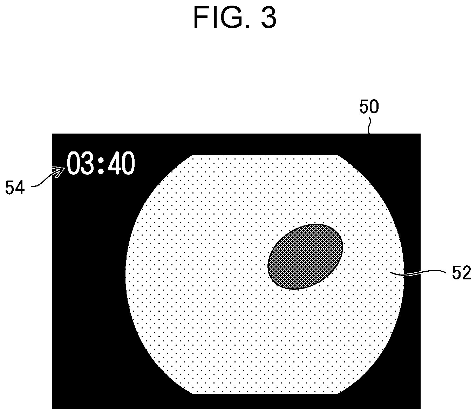

Fig. 3 is a diagram illustrating a first report example by using the display.

On a screen 50 of the display 16 illustrated in Fig. 3, a medical image 52 that is a moving image and character information 54 of "03:40" indicating a measured time (e.g., a current removal time) in a first display region of the display 16 are displayed. The first display region in the example is an upper left region of the screen 50 in Fig. 3, which is adjacent to the display region of the medical image 52.

In a non-measurement state (before start of measurement), the report processing unit 45 may display the character information 54 such as "00:00", or may hide or gray out the entirety of the displayed character information 54. Accordingly, the user can grasp the measurement state during the examination.

In addition, the report processing unit 45 causes the time measured by the time measurement processing unit 43 to be displayed in real time. From the character information 54 of the measured time that changes in real time, the user can grasp that the examination is being performed and the examination time from the start of the examination to the present.

Furthermore, by the character information 54 being changed from "00:00", or by the examination time being displayed from being hidden or grayed out, the user can grasp that the apparatus automatically recognizes the start of the examination and starts the time measurement.

In addition, by the change of the character information 54 stopping, or by the character information 54 being hidden or grayed out, the user can grasp that the apparatus automatically recognizes the end of the examination and ends the time measurement.

### Second Notification Example

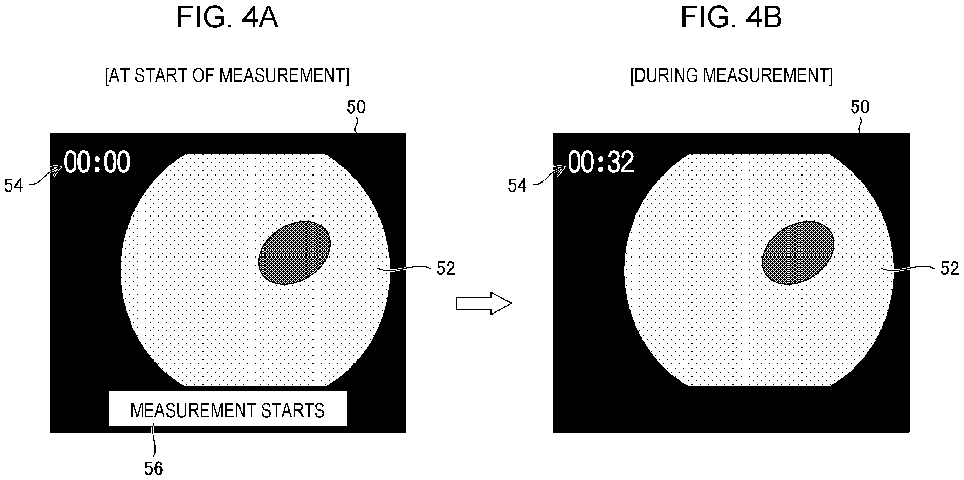

Figs. 4A and 4B are diagrams illustrating a second report example by using the display, Fig. 4A illustrates a screen of the display at the time of start of measurement, and Fig. 4B illustrates a screen of the display during measurement.

On the screen 50 of the display 16 illustrated in Figs. 4A and 4B, as in the first report example, the medical image 52 that is a moving image and the character information 54 of the measured time are displayed.

The report processing unit 45 further causes the display 16, which is the report unit, to report the information on the state of the time measurement to the user only for a certain period based on the timing at which the state of the time measurement is changed.

In the second report example illustrated in Figs. 4A, if the examination state recognition processing unit 42 recognizes a transition of the measurement state, for example, from non-measurement to the start of measurement, the report processing unit 45 causes the display 16 to display character information 56 of "measurement starts" as the information on the state of the time measurement only for a certain period from a measurement start time point.

Note that the report processing unit 45 causes the character information 56 to be displayed in a second display region (a display region adjacent to the lower side of the display region of the medical image 52 in this example) different from the first display region in which the character information 54 of the measured time is displayed.

The certain period during which the character information 56 of "measurement starts" is displayed is, for example, about several seconds, and is preferably a period during which the user can visually recognize the character information 56. Note that the certain period may be set by the user.

Since the user needs to closely observe the medical image 52 during the examination, the user tends not to pay attention to a region other than the medical image 52.

Thus, the character information 56 is preferably displayed to be larger than the character information 54 for reporting the measured time so that the user can easily notice the character information 56. On the other hand, if the character information 56 is reported in a noticeable manner, the examination may be interrupted. Thus, when the certain period elapses from the measurement start time point, the report of the character information 56 is ended as illustrated in Fig. 4B.

Accordingly, it is possible to report the information on the state of the time measurement in a more noticeable manner while suppressing an adverse effect on the examination. As a more noticeable display method, the state transition may be reported by the character information 56 as illustrated in Figs. 4A and 4B, or a symbol or the like may be used. In addition, the time that is being measured may be displayed together, or may not be displayed together.

In addition, the report processing unit 45 can report the information on the state of the time measurement to the user as sound information via the sound information reproduction processing unit 47 and the speaker 17 only for a certain period based on the timing at which the state of the time measurement is changed. For example, instead of the character information 56 of "measurement starts", or together with the character information 56 of "measurement starts", voice saying "measurement starts" can be output.

Note that the sound information is not limited to voice saying "measurement starts" or the like and may be a beep sound or the like for reporting that the state of the time measurement is changed.

Since the removal time or the treatment time in the endoscopic examination of the large intestine measured by the time measurement processing unit 43 needs to be left as an examination record, the CPU 41 stores the removal time or the treatment time in a storage unit (the memory 48) in association with a diagnostic report or the like.

At this time, for the CPU 41, a time measurement result measured only by the recognition result of the automatic recognition by the examination state recognition processing unit 42 and a time measurement result corrected by a user operation (e.g., a measurement result obtained when the user performs a measurement start operation because the examination state recognition processing unit 42 has made an error in the measurement start timing) are preferably stored in a form that can be distinguished from each other (storage methods of the time measurement results are preferably changed).

Note that the information on the state of the time measurement is information such as the examination time (removal time), the start of the examination, and the end of the examination in the first report example and the second report example illustrated in Figs. 3 and 4A and 4B, but is not limited to those and includes, for example, information such as a treatment time during a treatment using a treatment tool, the start of the treatment, and the end of the treatment.

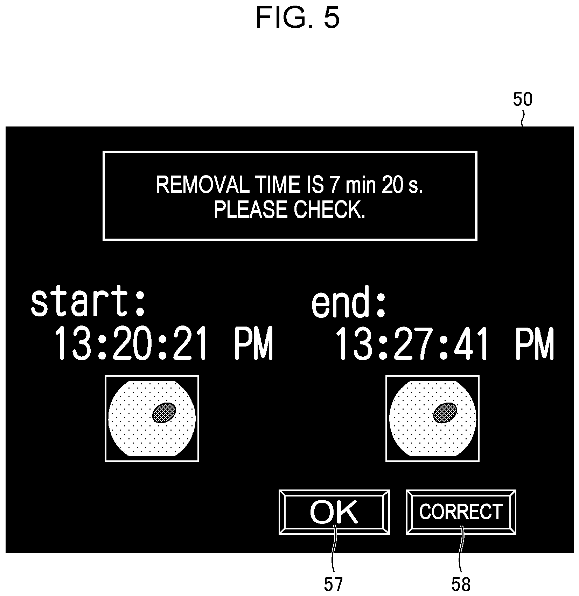

Fig. 5 is a diagram illustrating an example of a screen displayed on the display when the measurement result is stored.

Specifically, when the time information (X min Y sec) that is the measurement result is recorded, information of "no user correction" or "with user correction" is given to the measurement result and recorded. That is, if a behavior of the time measurement control processing (e.g., the start time point of the time measurement) is controlled by a user operation on the operating unit 15, the storage method of the time measurement result in the storage unit is changed in accordance with the situation of the controlled behavior.

Accordingly, the user can check the measurement result of "no user correction" after the end of the examination, and can later check whether there is a measurement error.

In addition, if recording is performed with "no user correction", display for prompting the user to check the measurement result is preferably performed before recording (immediately after the end of the examination).

Fig. 5 illustrates a display example for prompting the user to check the measurement result if recording is performed with "no user correction".

In the display example illustrated in Fig. 5, a message "The removal time is 7 min 20 s. Please check." for prompting the user to check the measurement result is displayed, and time information at the start or end of the measurement and a still image at that time are also displayed. Accordingly, the user can easily check whether the result of the automatic measurement is erroneous.

In addition, an "OK" button 57 and a "correction" button 58 are displayed. If the "OK" button 57 is selected, information of "no user correction" is added to the automatically measured measurement result, and the result is stored. If the "correction" button 58 is operated, information of "with user correction" is added to the corrected measurement result after correction of the measurement result by the user, and the result is stored.

The user can correct the measurement result by, for example, temporarily storing the moving image 38 with time stamps in the memory 48 and selecting a frame image at the start of the examination or a frame image at the end of the examination while reproducing the stored moving image 38.

Note that if the corrected measurement result is stored in the memory 48, the moving image 38 temporarily stored in the memory 48 can be deleted.

### Medical Image Processing Method

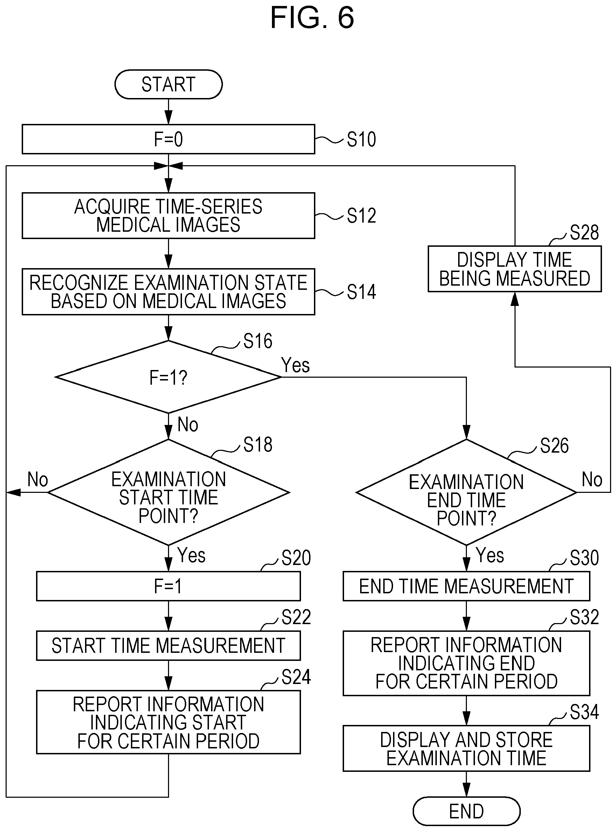

Fig. 6 is a flowchart illustrating an embodiment of a medical image processing method according to the present invention, and illustrates a processing procedure of each unit of the medical image processing apparatus 14 illustrated in Fig. 2.

In Fig. 6, the CPU 41 first sets a flag F indicating whether an examination is being performed, to F = 0, which indicates that the examination is not being performed (step S10). Subsequently, the medical image acquisition unit 40 acquires, from the endoscope processor apparatus 12, a current medical image that is a processing target (step S10).

The examination state recognition processing unit 42 recognizes the examination state on the basis of the medical image acquired in step S10 (including medical images acquired before the current time point) (step S12, examination state recognition step). For example, in the case of an endoscopic examination, whether an endoscope is being inserted, is being removed, or is outside a body is recognized, or whether a treatment is being performed with a treatment tool is recognized.

The CPU 41 determines whether the flag F is F = 1 (during examination), and if F = 1 is not satisfied (if "No"), the process proceeds to step S18, in which it is determined whether the current time point is an examination start time point. The examination start time point can be determined on the basis of the recognition result of the examination state obtained in step S14.

If it is determined in step S18 that the current time point is the examination start time point (if "Yes"), the flag F is set to F = 1 (during examination) (step S20), the time measurement control processing unit 44 causes the time measurement processing unit 43 to start the time measurement, and the time measurement processing unit 43 measures the examination time (step S22, time measurement control step and time measurement step).

Subsequently, the report processing unit 45 causes the display 16 and/or the speaker 17 to report information indicating the start of the examination only for a certain period based on the timing of the start of the examination, and then transitions to step S12 (step S24, report step). In the report example illustrated in Figs. 4A and 4B, the display 16 is caused to display the character information 56 of "measurement starts" (Fig. 4A), and when the certain period elapses, the character information 56 is deleted, and the character information 54 of the measured time is continuously displayed.

On the other hand, when the examination starts, since the flag F is set to F = 1 (during examination), it is determined in step S16 that the examination is being performed, and the process proceeds to step S26.

In step S26, it is determined whether the current time point is an examination end time point. The examination end time point can be determined on the basis of the recognition result of the examination state obtained in step S14.

If it is determined in step S26 that the current time point is the examination end time point (if "No"), the display 16 is caused to display the measured time during the examination (during measurement), and the process returns to step S12 (step S28).

On the other hand, if it is determined in step S26 that the current time point is the examination end time point (if "Yes"), the time measurement control processing unit 44 causes the time measurement processing unit 43 to end the time measurement (step S30), and the report processing unit 45 causes the display 16 and/or the speaker 17 to report information indicating the end of the examination only for a certain period based on the timing of the end of the examination (step S32, report step).

Subsequently, the display 16 is caused to display the examination time (removal time) of the endoscopic examination or the treatment time measured by the time measurement processing unit 43 (see Fig. 5), and is stored in the memory 48 in association with a diagnostic report or the like (step S34).

Note that the determination of the examination start time point in step S18 in Fig. 6 and the determination of the examination start time point in step S26 are performed on the basis of the recognition result of the examination state obtained by the examination state recognition processing unit 42, but the examination start time point and the examination start time point may also be determined on the basis of a user instruction by a user operation on the operating unit 15. In a case where the user issues an instruction of the examination start time point or the examination start time point, instead of the recognition result obtained by the examination state recognition processing unit 42, it is preferable that the examination start time point or the examination end time point according to the user instruction be preferentially used, and the start and end of the time measurement of the examination time be controlled.

### Miscellaneous

Although the endoscope processor apparatus and the medical image processing apparatus are separately provided in the above embodiment, the endoscope processor apparatus and the medical image processing apparatus may be integrated. That is, the endoscope processor apparatus may be provided with the functions of the medical image processing apparatus.

In addition, the measured examination time or treatment time is stored in the memory within the medical image processing apparatus in association with a diagnosis report or the like, but is not limited to this and may also be stored in an external memory (storage unit) connected to the medical image processing apparatus.

Furthermore, the medical image is not limited to an endoscopic image captured by an endoscope and may be, for example, time-series images acquired by another modality such as an ultrasound diagnostic apparatus.

In addition, a hardware configuration that performs various controls of the medical image processing apparatus according to the above embodiment is any of the following various processors. Various processors include a central processing unit (CPU), which is a general-purpose processor that executes software (program) and functions as various control units, a programmable logic device (PLD), which is a processor in which the circuit configuration is changeable after manufacture, such as field programmable gate array (FPGA), a dedicated electric circuit, which is a processor having a circuit configuration that is specially designed to execute various kinds of processing, such as an application specific integrated circuit (ASIC), and the like.

One processing unit may be constituted by one of these various processors, or may be constituted by two or more processors of the same type or different types (e.g., a combination of a plurality of FPGAs or a combination of a CPU and an FPGA). In addition, a plurality of control units may be constituted by one processor. As examples for constituting a plurality of control units by one processor, firstly, there is a form in which one or more CPUs and software are combined to constitute one processor, and this processor functions as a plurality of control units, as typified by a computer such as a client or a server. Secondly, there is a form of using a processor that implements the functions of the entire system including a plurality of control units by using one integrated circuit (IC) chip, as typified by a system on chip (SoC) or the like. In this manner, various control units are constituted by one or more of the above various processors in terms of hardware configuration.

Furthermore, the present invention includes a medical image processing program to be installed in a computer to cause the computer to function as the medical image processing apparatus according to the present invention, and a non-volatile storage medium in which the medical image processing program is recorded.

Furthermore, the present invention is not limited to the above-described embodiment, and it is needless to say that various modifications can be made without departing from the spirit of the present invention.

### Reference Signs List

- 9: endoscope system
- 10: endoscope
- 11: light source apparatus
- 12: endoscope processor apparatus
- 13: display apparatus
- 14: medical image processing apparatus
- 15: operating unit
- 16: display
- 17: speaker
- 20: insertion part
- 21: handheld operating unit
- 22: universal cord
- 25: soft part
- 26: bending part
- 27: tip part
- 28: imaging element
- 29: bending operation knob
- 30: air/water supply button
- 31: suction button
- 32: still image capturing instruction unit
- 33: treatment tool introduction port
- 35: light guide
- 36: signal cable
- 37a, 37b: connector
- 38: moving image
- 38a: frame image
- 39: still image
- 40: medical image acquisition unit
- 41: CPU
- 42: examination state recognition processing unit
- 43: time measurement processing unit
- 44: time measurement control processing unit
- 45: report processing unit
- 46: display control unit
- 46A: image display control unit
- 46B: information display control unit
- 47: sound information reproduction processing unit
- 48: memory
- 50: screen
- 52: medical image
- 54, 56: character information
- 57: "OK" button
- 58: "correction" button
- F: flag
- S10 to S34: step

## Claims

1. A medical image processing apparatus comprising a processor configured to perform:
medical image acquisition processing for sequentially acquiring time-series medical images;
examination state recognition processing for recognizing an examination state on the basis of the sequentially acquired medical images;
time measurement processing for performing time measurement related to the examination state;
time measurement control processing for controlling a behavior of the time measurement in accordance with a recognition result of the examination state; and
report processing for causing a report unit to report information on a state of the time measurement to a user.

2. The medical image processing apparatus according to claim 1, wherein, in the report processing, the report unit is caused to report the information on the state of the time measurement to the user only for a certain period based on a timing at which the state of the time measurement is changed.

3. The medical image processing apparatus according to claim 1 or 2, wherein, in the report processing, a display unit functioning as the report unit is caused to display information on a time measured in the time measurement processing in real time in a first display region of the display unit and to display the information on the state of the time measurement in a second display region of the display unit, the second display region being different from the first display region, or a speaker functioning as the report unit is caused to report the information on the state of the time measurement as sound information.

4. The medical image processing apparatus according to any one of claims 1 to 3, wherein
the information on the state of the time measurement includes information of one or more of a start, an end, a pause, or a restart of the time measurement, and
in the time measurement control processing, control of one or more of the start, the end, the pause, or the restart of the time measurement in the time measurement processing is performed.

5. The medical image processing apparatus according to any one of claims 1 to 4, wherein,
the processor is configured to perform reception processing for receiving the information on the state of the time measurement from a user operating unit, and
in the time measurement control processing, upon reception of the information on the state of the time measurement from the user operating unit, the behavior of the time measurement is controlled by preferentially using the received information on the state of the time measurement instead of the recognition result obtained in the examination state recognition processing.

6. The medical image processing apparatus according to any one of claims 1 to 5, wherein the processor is configured to perform processing for storing, in a storage unit, a time measurement result obtained in the time measurement processing.

7. The medical image processing apparatus according to claim 5, wherein the processor is configured to perform processing for storing, in a storage unit, a time measurement result obtained in the time measurement processing, and, if a behavior of the time measurement control processing is controlled by a user operation on the user operating unit, change a method for storing the time measurement result in the storage unit in accordance with a situation of the controlled behavior.

8. The medical image processing apparatus according to any one of claims 1 to 7, wherein, in the examination state recognition processing, whether the examination state indicates that a treatment is being performed is recognized on the basis of the sequentially acquired medical images.

9. The medical image processing apparatus according to claim 8, wherein, in the examination state recognition processing, treatment tool detection processing for detecting a treatment tool is performed on the basis of the sequentially acquired medical images, and whether the treatment is being performed is recognized in accordance with a detection result of the treatment tool.

10. The medical image processing apparatus according to any one of claims 1 to 9, wherein the medical images are endoscopic images.

11. The medical image processing apparatus according to claim 10, wherein, in the examination state recognition processing, whether an endoscope is being inserted, is being removed, or is outside a body is recognized on the basis of the sequentially acquired medical images.

12. The medical image processing apparatus according to claim 10 or 11, wherein, in the examination state recognition processing, landmark detection processing for detecting a landmark in a lumen is performed on the basis of the sequentially acquired medical images, and the examination state is recognized in accordance with a detection result of the landmark.

13. The medical image processing apparatus according to claim 11 or 12, wherein, in the examination state recognition processing, movement detection processing for detecting a movement of the endoscope is performed on the basis of the sequentially acquired medical images, and the examination state is recognized in accordance with a detection result of the movement of the endoscope.

14. A medical image processing method comprising:
a step of sequentially acquiring time-series medical images;
an examination state recognition step of recognizing information on an examination state on the basis of the sequentially acquired medical images;
a time measurement step of performing time measurement related to the examination state;
a time measurement control step of controlling a behavior of the time measurement in accordance with a recognition result of the examination state; and
a report step of causing a report unit to report information on a state of the time measurement to a user, wherein
a processor is configured to execute processing in each step.

15. The medical image processing method according to claim 14, wherein, in the report step, the report unit is caused to report the information on the state of the time measurement to the user only for a certain period based on a timing at which the state of the time measurement is changed.

16. The medical image processing method according to claim 14 or 15, wherein, in the report step, a display unit functioning as the report unit is caused to display information on a time measured in the time measurement step in real time in a first display region of the display unit and to display the information on the state of the time measurement in a second display region of the display unit, the second display region being different from the first display region, or a speaker functioning as the report unit is caused to report the information on the state of the time measurement as sound information.

17. The medical image processing method according to any one of claims 14 to 16, comprising a step of receiving the information on the state of the time measurement from a user operating unit, wherein
in the time measurement control step, upon reception of the information on the state of the time measurement from the user operating unit, the behavior of the time measurement is controlled by preferentially using the received information on the state of the time measurement instead of the recognition result obtained in the examination state recognition step.

18. A medical image processing program for causing the processor to execute processing in each step in the medical image processing method according to any one of claims 14 to 17.

19. A non-transitory computer-readable recording medium on which the program according to claim 18 is recorded.
